# EUROPEAN PATENT APPLICATION

(11) **EP 3 649 992 A1**
(43) Date of publication of application: **13.05.2020**
(21) Application number: 19207021.7
(22) Date of filing: 05.11.2019
(51) Int. Cl.: A61F 5/01

(54) **DEVICE FOR TREATING DYSARTHROSIS**

(30) Priority: 08.11.2018 TW 107139716
(71) Applicant: Shih, Wan-Hsi, 403 Taichung City (TW)
(72) Inventor: Shih, Wan-Hsi, 403 Taichung City (TW)
(74) Representative: Lang, Christian

(57) **Abstract**

A device for treating dysarthrosis includes a first base having one side surface for the heel of a human foot to be attached thereon; a second base having one end disposed on the side surface of the first base and one side surface for the sole of the human foot to be attached thereon; a push portion disposed on the second base and having an abutting surface corresponding in position to an outer side of the first toe of the human foot and configured to abut against an outer side of the first metatarsophalangeal joint of the first toe to be moved between a first position and a second position; and a support portion disposed on the second base and having one end located between the first toe and the second toe of the human foot for abutting against one side of the first toe.

## Description

### FIELD OF THE INVENTION

The present invention relates to a device for treating dysarthrosis, and more particularly to a device for treating a deformity of the first metatarsophalangeal joint of the big toe of a human foot.

### BACKGROUND OF THE INVENTION

Hallux Valgus (HV) is a common foot deformity in which the big toe (the Hallux) deviates into the lateral or outer side of the foot (valgus deviation). The first toe slants toward the second toe, that is, the first metatarsophalangeal joint of the big toe protrudes outwardly. Most of Hallux Valgus is due to congenital inheritance or abnormal use of the foot, especially for a person who wears high heels or unfit shoes for a long time. As a result, when walking, the whole body weight falls on the front end of the foot, affecting the function of the foot arch. This causes the first toe and other toes to gradually deform due to improper compression, leading to a deformity of the first metatarsophalangeal joint of the big toe, namely, Hallux Valgus. The deformed first metatarsophalangeal joint often rubs against shoes, which leads to broken skin, calluses and corns. When the symptoms are severe, orthopedics or surgery is needed.

In general, the user wears a Hallux Valgus brace located between the first toe and the second toe for treating Hallux Valgus. The brace is a cylinder made of a soft material such as silicone. The cylinder is provided with a clamping portion having a recess. The brace is placed between the first toe and the second toe to prop the valgus big toe (first toe). However, in this way, it is not easy to treat the deformed first metatarsophalangeal joint to the normal position in a short time.

### SUMMARY OF THE INVENTION

The primary aspect of the present invention is to provide a device for treating dysarthrosis, which can treat a deformity of the first metatarsophalangeal joint of the big toe effectively in a short time.

Another aspect of the present invention is to provide a device for treating dysarthrosis, which provides a noninvasive treatment.

In order to achieve the above objects, a device for treating dysarthrosis is provided. The device for treating dysarthrosis comprises a first base, a second base, a push portion, and a support portion. The first base has one side surface for the heel of a human foot to be attached thereon. An angle is defined between the second base and the first base. One end of the second base is disposed on the side surface of the first base. The second base has one side surface for the sole of the human foot to be attached thereon. The push portion is disposed on the second base and has an abutting surface at one end thereof. The abutting surface corresponds in position to an outer side of the first toe of the human foot and is configured to abut against an outer side of the first metatarsophalangeal joint of the first toe to be moved between a first position and a second position. The support portion is disposed on the second base. One end of the support portion is located between the first toe and the second toe of the human foot for abutting against one side of the first toe.

Embodiments of the present invention will now be described, by way of example only, with reference to the accompanying drawings.

### BRIEF DESCRIPTION OF THE DRAWINGS

FIG. 1 is a perspective view in accordance with a first embodiment of the present invention;
FIG. 2 is a schematic view of FIG. 1 seen in another direction;
FIG. 3 is an exploded view of FIG. 2;
FIG. 4 is a schematic view in accordance with the first embodiment of the present invention when in use;
FIG. 5 is a perspective view of a support portion in accordance with a second embodiment of the present invention;
FIG. 6 is an exploded view of the support portion of FIG. 5;
FIG. 7 is a schematic view of FIG. 5 seen in another direction;
FIG. 8 is a schematic view of FIG. 6 seen in another direction;
FIG. 9 is a perspective view in accordance with a third embodiment of the present invention; and
FIG. 10 is a partially exploded view of FIG. 9.

### DETAILED DESCRIPTION OF THE PREFERRED EMBODIMENTS

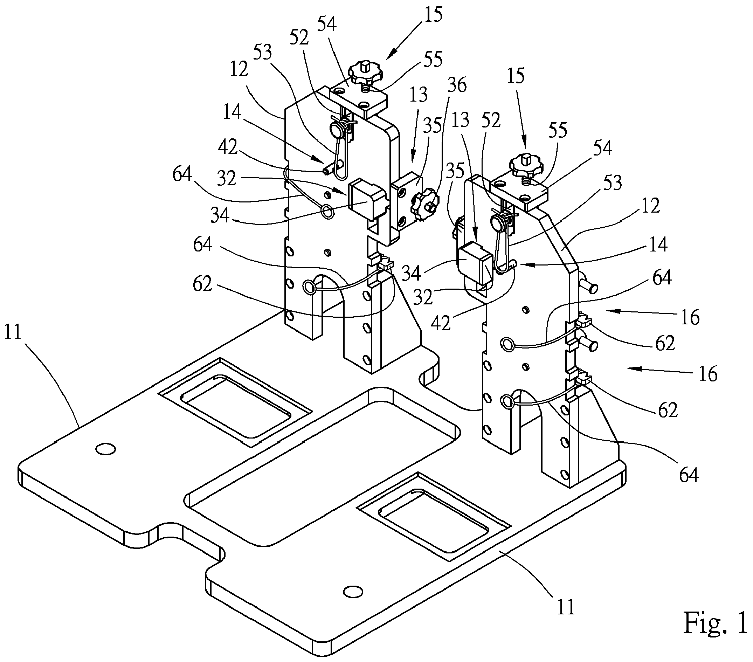

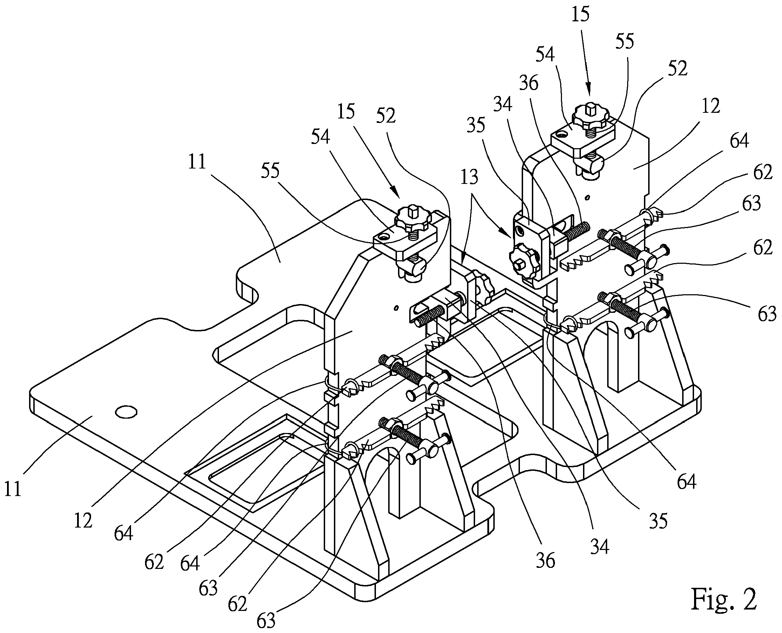

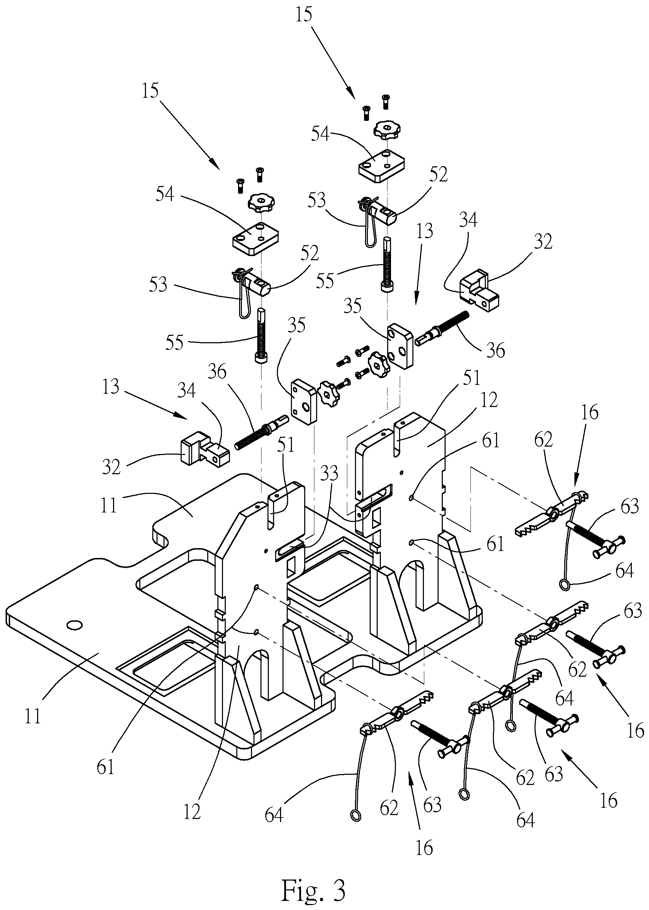

Referring to FIG. 1 to FIG. 4, the present invention discloses a device for treating dysarthrosis, comprising a first base 11, a second base 12, a push portion 13, and a support portion 14.

The first base 11 has one side surface for the heel 10 of a human foot to be attached thereon.

An angle is defined between the second base 12 and the first base 11. One end of the second base 12 is disposed on the side surface of the first base 11. The second base 12 has one side surface for the sole 20 of the human foot to be attached thereon.

The push portion 13 is disposed on the second base 12 and has an abutting surface 32 at one end thereof. The abutting surface 32 corresponds in position to the outer side of the first toe 30 of the human foot, and is configured to abut against the outer side of the joint between the first toe 30 and the sole 20 (the first metatarsophalangeal joint of the first toe) to be moved between a first position and a second position.

The push portion 13 includes a groove 33, a slider 34, a bracket 35, and a screw 36. The groove 33 is defined in the second base 12. A middle portion of the slider 34 is movably inserted in the groove 33. The abutting surface 32 is disposed on one end of the slider 34. The bracket 35 is fixed to the second base 12. A middle portion of the screw 36 is rotatably inserted through a perforation of the bracket 35. One end of the screw 36 is provided with an external thread and inserted into an opening having an internal thread at another end of the slider 34. When another end of the screw 36 is rotated, the slider 34 is driven to move between two positions being respectively corresponding to the first position and the second position.

The support portion 14 includes a post 42 and is disposed on the second base 12. One end of the support portion 14 is located between the first toe 30 and the second toe 40 of the human foot for abutting against one side of the first toe 30 when the first toe 30 is pushed.

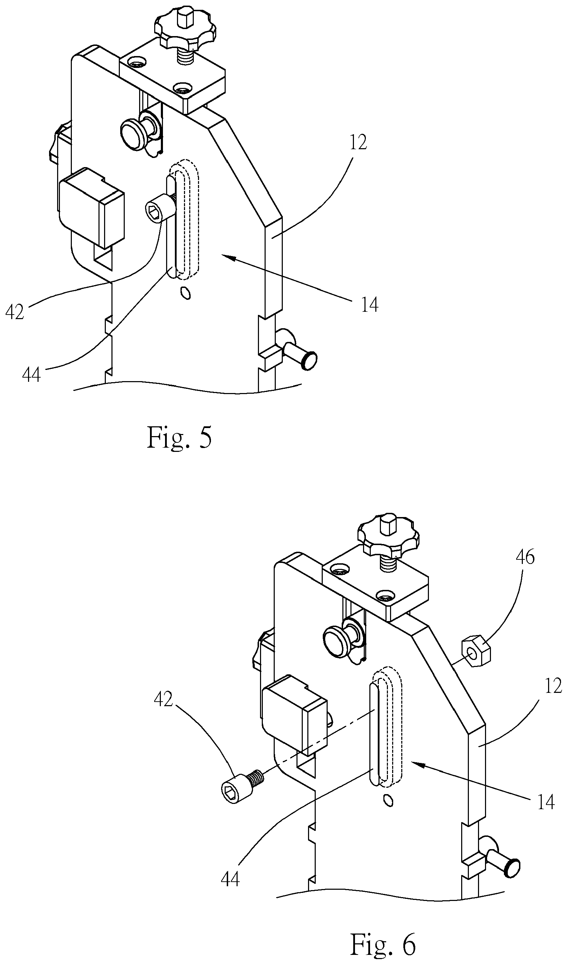

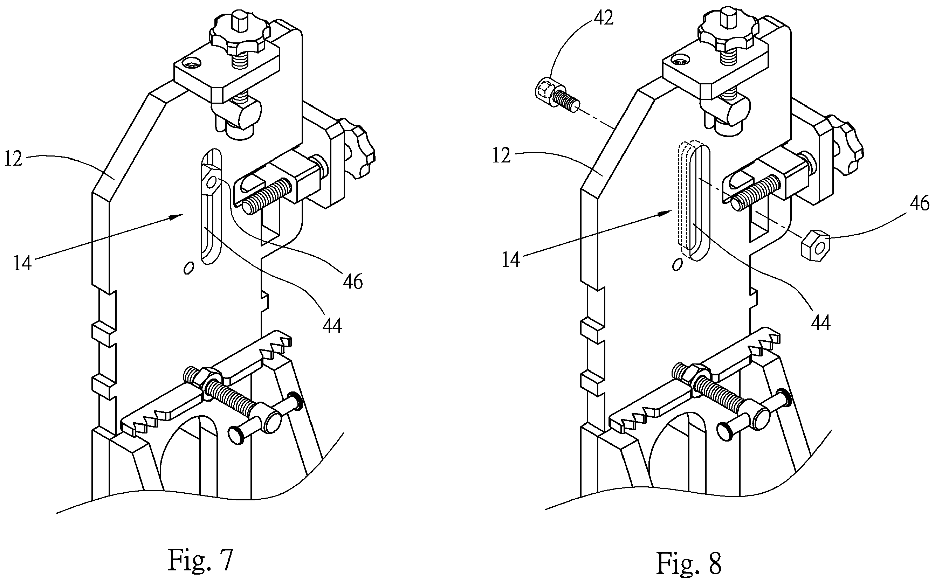

Referring to FIG. 5 through FIG. 8, the support portion 14 further includes a slot 44 and a nut 46. The slot 44 is defined in the second base 12. One end of the post 42 is disposed on one side of the second base 12 and is located between the first toe 30 (not shown) and the second toe 40 (not shown) of the human foot. Another end of the post 42 has an external thread and is inserted through the slot 44 to be connected with the nut 46 disposed on another side of the second base 12. The nut 46 is configured to rotate the post 42 to move and position the post 42 on the second base 12. The nut 46 can be movably but non-rotatably inserted into the slot 44 for conveniently and instantly changing the position of the post 42 as long as the post 42 is rotated, depending on the size of the human foot.

When the first metatarsophalangeal joint of the first toe of the human foot is in an abnormal position (so-called Hallux Valgus), the present invention provides a function for treating Hallux Valgus.

Referring to FIG. 1, FIG. 3 and FIG. 4, the present invention further comprises a traction portion 15. The traction portion 15 is disposed on the second base 12 by its one end being corresponding to the outer end of the first toe 30 of the human foot. One end of the traction portion 15 is attached to the first toe 30 to generate a pulling force on the first toe 30, so that the invention has a more effective function for treating Hallux Valgus.

The traction portion 15 includes a groove 51, a slider 52, a connecting strap 53, a bracket 54 and a screw 55. The groove 51 is defined in the second base 12. A middle portion of the slider 52 is movably inserted through the groove 51. One end of the connecting strap 53 is attached to one end of the slider 52, and another end of the connecting strap 53 is attached to the first toe 30. The bracket 54 is fixed to the second base 12. A middle portion of the screw 55 is rotatably inserted through a perforation of the bracket 54. One end of the screw 55 is provided with an external thread and inserted into an opening having an internal thread at another end of the slider 52. When another end of the screw 55 is rotated, the slider 34 is driven to move between a third position and a fourth position to generate a pulling force on the first toe 30 through the connecting strap 53.

Referring to FIG. 1 through FIG. 4, the present invention further comprises a stabilizing portion 16. The stabilizing portion 16 is disposed on the other side surface of the second base 12 corresponding to the sole 20 of the human foot. One end of the stabilizing portion 16 is attached to the instep 50 of the human foot to stabilize the human foot.

The stabilizing portion 16 includes a through hole 61, a movable block 62, a screw 63, and a connecting strap 64. The through hole 61 is defined in the second base 12 and corresponds to the sole of the human foot. The movable block 62 corresponds to the sole 20 of the human foot and is located on the other side surface of the second base 12. One end of the screw 63 is rotatably inserted in the through hole 61. A middle portion of the screw 63 is provided with an external thread and is inserted through an opening having an internal thread at a middle portion of the movable block 62. When another end of the screw 63 is rotated, the movable block 62 is driven to move between a fifth position and a sixth position. Two ends of the connecting strap 64 are attached to two ends of the movable block 62, respectively. A middle portion of the connecting strap 64 is attached to the instep 50 of the human foot for generating a pulling force on the human foot by the movement of the movable block 62 to stabilize the human foot.

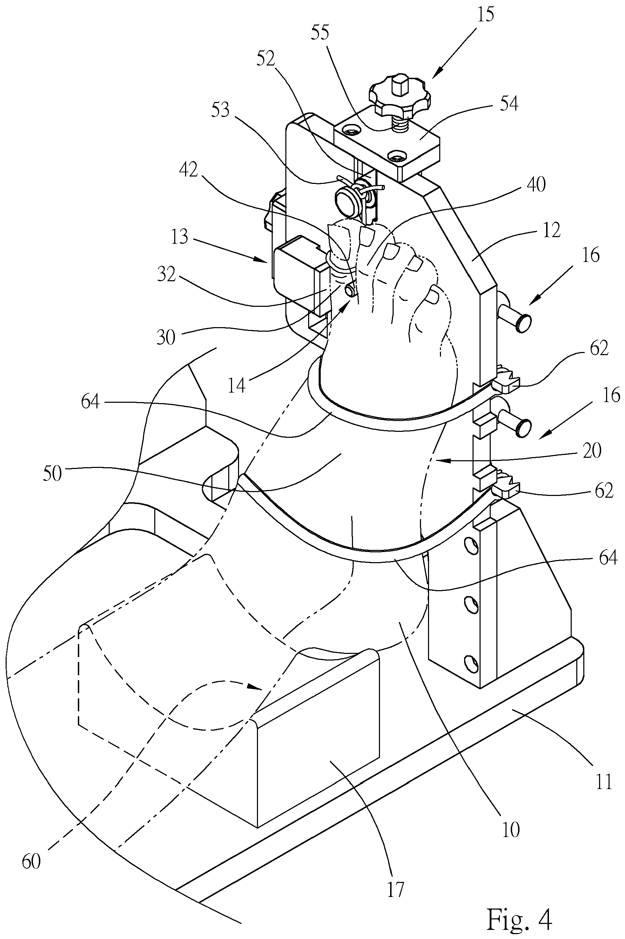

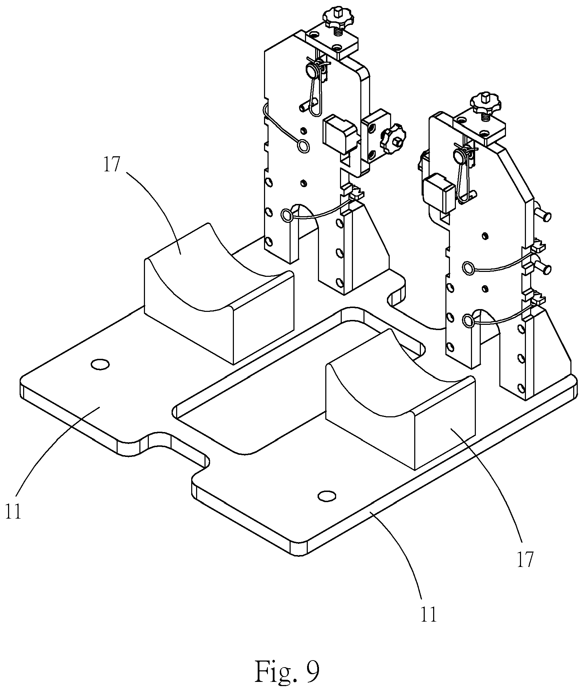

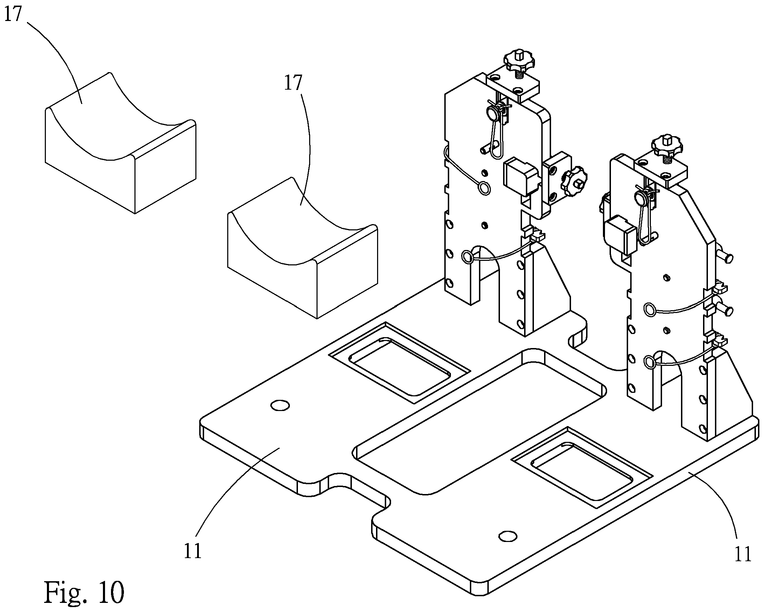

Referring to FIG. 4, FIG. 9 and FIG. 10, the present invention further comprises a compensating portion 17. The compensating portion 17 is disposed between the outer side of Achilles tendon 60 of the human foot and the first base 11 for compensating the gap between the outer side of the Achilles tendon 60 of the human foot and the first base 11, thereby providing better comfort and stability to the human foot. The compensating portion 17 may be a block having an arcuate recess.

In the device for treating dysarthrosis disclosed in the present invention, the abutting surface 32 and the post 42 are formed of a soft material such as rubber or coated with a soft material such as rubber for better comfort of the human foot in the treatment of Hallux Valgus.

The device for treating dysarthrosis of the present invention may be a pair of mutually symmetrical structures for left and right human feet.

The above embodiments are only used to illustrate the present invention, not intended to limit the scope thereof. The scope of the claims should not be limited by the preferred embodiments set forth in the examples, but should be given the broadest interpretation consistent with the description as a whole.

## Claims

1. A device for treating dysarthrosis, comprising:
a first base, having one side surface for the heel of a human foot to be attached thereon;
a second base, an angle being defined between the second base and the first base, one end of the second base being disposed on the side surface of the first base, the second base having one side surface for the sole of the human foot to be attached thereon;
a push portion, disposed on the second base and having an abutting surface at one end thereof, the abutting surface corresponding in position to an outer side of the first toe of the human foot and being configured to abut against an outer side of the first metatarsophalangeal joint of the first toe to be moved between a first position and a second position; and
a support portion, disposed on the second base, one end of the support portion being located between the first toe and the second toe of the human foot for abutting against one side of the first toe.

2. The device as claimed in claim 1, further comprising a traction portion;
the traction portion being disposed on the second base by its one end being corresponding to an outer end of the first toe of the human foot, one end of the traction portion being attached to the first toe to generate a pulling force on the first toe.

3. The device as claimed in claim 1, further comprising a stabilizing portion;
the stabilizing portion being disposed on another side surface of the second base corresponding to the sole of the human foot, one end of the stabilizing portion being attached to the instep of the human foot to stabilize the human foot.

4. The device as claimed in claim 2, further comprising a stabilizing portion;
the stabilizing portion being disposed on another side surface of the second base corresponding to the sole of the human foot, one end of the stabilizing portion being attached to the instep of the human foot to stabilize the human foot.

5. The device as claimed in claim 1, further comprising a compensating portion;
the compensating portion being disposed between an outer side of Achilles tendon of the human foot and the first base for compensating a gap between the outer side of the Achilles tendon of the human foot and the first base.

6. The device as claimed in claim 2, further comprising a compensating portion;
the compensating portion being disposed between an outer side of Achilles tendon of the human foot and the first base for compensating a gap between the outer side of the Achilles tendon of the human foot and the first base.

7. The device as claimed in claim 3, further comprising a compensating portion;
the compensating portion being disposed between an outer side of Achilles tendon of the human foot and the first base for compensating a gap between the outer side of the Achilles tendon of the human foot and the first base.

8. The device as claimed in claim 4, further comprising a compensating portion;
the compensating portion being disposed between an outer side of Achilles tendon of the human foot and the first base for compensating a gap between the outer side of the Achilles tendon of the human foot and the first base.

9. The device as claimed in any one of claims 1 to 8, wherein the push portion includes a groove, a slider, a bracket, and a screw;
the groove is defined in the second base;
a middle portion of the slider is movably inserted in the groove, the abutting surface is disposed on one end of the slider; the bracket is fixed to the second base;
a middle portion of the screw is rotatably inserted through a perforation of the bracket, one end of the screw is provided with an external thread and inserted into an opening having an internal thread at another end of the slider, when another end of the screw is rotated, the slider is driven to move between two positions being respectively corresponding to the first position and the second position.

10. The device as claimed in any one of claims 1 to 8, wherein the support portion includes a slot, a post, and a nut;
the slot is defined in the second base;
one end of the post is disposed on one side of the second base and is located between the first toe and the second toe of the human foot, another end of the post has an external thread and is inserted through the slot to be connected with the nut disposed on another side of the second base, and the nut is configured to rotate the post to move and position the post on the second base.

11. The device as claimed in claim 2, 4, 6 or 8, wherein the traction portion includes a groove, a slider, a connecting strap, a bracket, and a screw;
the groove is defined in the second base;
a middle portion of the slider is movably inserted through the groove;
one end of the connecting strap is attached to one end of the slider, another end of the connecting strap is attached to the first toe;
the bracket is fixed to the second base;
a middle portion of the screw is rotatably inserted through a perforation of the bracket, one end of the screw is provided with an external thread and inserted into an opening having an internal thread at another end of the slider, when another end of the screw is rotated, the slider is driven to move between a third position and a fourth position to generate a pulling force on the first toe through the connecting strap.

12. The device as claimed in claim 3, 4, 7 or 8, wherein the stabilizing portion includes a through hole, a movable block, a screw, and a connecting strap;
the through hole is defined in the second base and corresponds to the sole of the human foot;
the movable block corresponds to the sole of the human foot and is located on another side surface of the second base;
one end of the screw is rotatably inserted in the through hole, a middle portion of the screw is provided with an external thread and is inserted through an opening having an internal thread at a middle portion of the movable block, when another end of the screw is rotated, the movable block is driven to move between a fifth position and a sixth position;
two ends of the connecting strap are attached to two ends of the movable block respectively, and a middle portion of the connecting strap is attached to the instep of the human foot for generating a pulling force on the human foot by movement of the movable block.
